# EUROPEAN PATENT SPECIFICATION

(11) **EP 0 571 983 B1**
(45) Date of publication and mention of the grant of the patent: **08.04.1998**
(21) Application number: 93108513.8
(22) Date of filing: 26.05.1993
(51) Int. Cl.: C07C 69/54, A61K 6/083, C07C 67/26, C08G 59/42, C08G 59/14

(54) **Alpha, omega-acrylate terminated macromonomer compounds**
Alpha, omega-acrylat terminierte, makromonomerische Verbindungen
Composés macromonomériques terminés en alpha, oméga avec acrylate

(30) Priority: 29.05.1992 DE 4217761
(43) Date of publication of application: 01.12.1993
(73) Proprietor: DENTSPLY GMBH, D-78467 Konstanz (DE)
(72) Inventor: Klee, Joachim E., W-7760 Radolfzell (DE); Horhold, Hans-Heinrich, O-6902 Jena (DE); Clauben, Frank, Dr., O-6902 Jena (DE)
(74) Representative: Wächtershäuser, Günter, Prof. Dr.

(56) References cited:
- EP-A- 0 104 491
- EP-A- 0 188 752
- DD-A- 277 689
- US-A- 3 066 112
- US-A- 3 256 226
- US-A- 4 097 569
- CHEMICAL ABSTRACTS, vol. 89, no. 18, 30 October 1978, Columbus, Ohio, US; abstract no. 148211c, page 71 ;column 2 ; & LAKOKRAS. MATER. IKH. PRIMEN., no.4, 1978 pages 50 - 52

## Description

The invention relates to compounds having repetitive units of α,ω-acrylate terminated macromonomer epoxide-carboxylic acid. Synthesis of such compounds is carried out by a two-step reaction or by simultaneous polymerization of diepoxides, dicarboxylic acids and unsaturated monocarboxylic acids. These compounds are useful as dental adhesives, dental cements, dental restoratives and dental luting materials.

α,ω-(meth)acrylate terminated epoxide-amine macromonomers recently described in patent application (DD 279667) are not comparable to the α,ω-methacrylate epoxide-carboxylic acid macromonomers of the present invention. Nor is the synthesis of the α,ω-methacrylate terminated epoxide carboxylic acid macromonomers of the present invention comparable to the synthesis of amine-macromonomers, because carboxylic acids react at higher temperatures with epoxides than do amines (see Houben-Weyl Bd 14/2, S. 499ff). At such high reaction temperatures there is concern that the polymerization of the unsaturated monocarboxylic acid may occur. M.Fedkte et al, in Plaste und Kautschuk 31 (1984) 405; K. Dusek, L. Matejka, Amer. Chem. Soc. 1984, 15, disclose reaction of epoxide-carboxylic acid to form insoluble network polymers caused by side reactions of epoxides and hydroxylic groups wherein R is an aromatic diepoxide moiety. The synthesis of α,ω-methacrylate terminated epoxide carboxylic acid macromonomers in accordance with the present invention is surprising. EP-A-0 104 491 describes α,ω-terminated monoepoxide-ester macromonomers.

As used herein "(Meth)acrylic acid" is understood to mean acrylic and methacrylic acids.

### Brief Summary of the Invention

The invention provides α,ω-acrylate terminated epoxide-carboxylic acid macromonomer compounds formed by reaction of at least one diepoxide, at least one dicarboxylic acid and at least one unsaturated mono-carboxylic acid and having the general formula I: 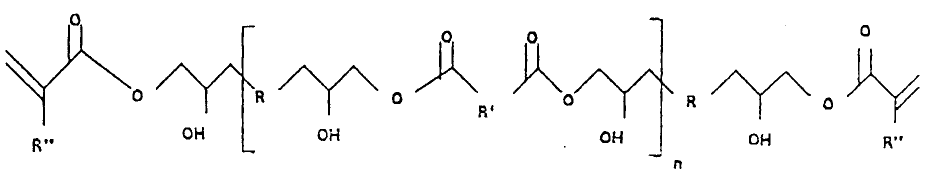
wherein R is on aromatic moiety formed from a diepoxide,
R' is a substituted or unsubstituted aliphatic, araliphatic, cycloaliphatic or aromatic moiety formed from a dicarboxylic acid,
R'' is hydrogen, a substituted or unsubstituted aliphatic, araliphatic, aromatic or cycloaliphatic moiety formed from a mono-carboxylic acid, and n an integer from 1 to 20.

### Detailed description of the invention

The invention provides α,ω-acrylate terminated epoxide-carboxylic acid macromonomer compounds within the scope of general formula (I): 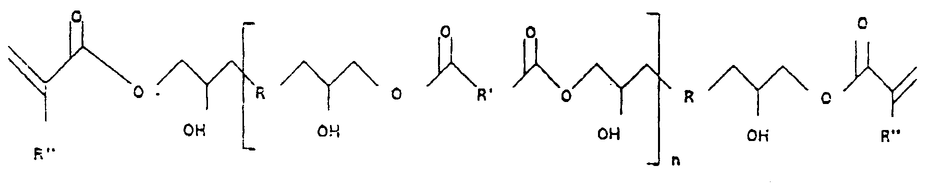
wherein R is a residue arisen from a diepoxide, such as 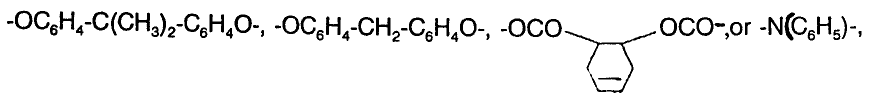
R' is a difunctional substituted or unsubstituted aliphatic, araliphatic, cycloaliphatic or aromatic residue, such as
   -CH₂CH₂-, -CH₂CH₂CH₂CH₂-, -CH₂-,-(CH₂)₃-, -C₆H₄-, or -C₆H₁₀-,
R'' is hydrogen or a monofunctional substituted or unsubstitued aliphatic, araliphatic, aromatic or cycloaliphatic moiety, but preferably CH₃ , and
n is an integer from 1 to 20. Compounds within the scope of general formula I are prepared by reaction of diepoxides, dicarboxylic acids and unsaturated monocarboxylic acids. Preferably this reaction is carried out in the presence of catalysts and/or accelerators near or in solvents such as tetrahydrofuran, toluene, or triethyleneglycoldimethacrylate at temperatures between 60 to 150°C.

In accordance with the invention are provided compositions which include at least one compound within the scope of general formula I and at least one polymerizable low molecular weight oligomer having a moiety formed from at least one diepoxide and at least one monocarboxylic acid.

Methacrylic acid is a preferred monocarboxylic acid used for the synthesis of α,ω-methacrylate terminated epoxide-carboxylic acid macromonomers of formula 2: 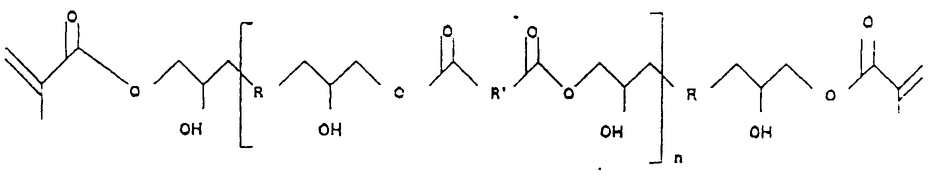
wherein R is a residue arisen from a diepoxide, such as 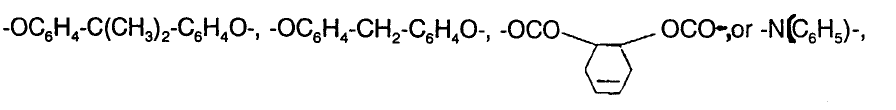
R' is a difunctional substituted or unsubstituted aliphatic, araliphatic, cycloaliphatic or aromatic residue, such as
   -CH₂CH₂-, -CH₂CH₂CH₂CH₂-, -CH₂-,-(CH₂)₃-, -C₆H₄-, or -C₆H₁₀-,
n is an integer from 1 to 20,

The α,ω-acrylate terminated epoxide-carboxylic acid-macromonomers are preferably prepared in two steps. In the first step a prepolymer is formed by reaction of diepoxides with dicarboxylic acids. Then these prepolymers are terminated by reaction with an unsaturated monocarboxylic acid. The reactions in these steps may preferably occur simultaneously. Preferably tetrahydrofuran, dioxane, and/or toluene, are used as solvents for these reactants.

Polymerizable diluents such as triethyleneglycolbismethacrylate, diethyleneglycolbismethacrylate, dioxolanbismethacrylate, vinyl-, vinylene- or vinylidene-compounds, acrylate or methacrylate substituted spiroorthoesters, ethoxylated Bis-GMA, are preferably used as solvents for the synthesis of macromonomers in accordance with the invention. Thus polymerizable diluents preferably became part of a macromonomer containing solution to provide a useful function such as reduced viscosity desirable to compound dental composites for restorative dentistry.

The α,ω-acrylate terminated epoxide-carboxylic acid macromonomers within the scope of general formulas 1 and 2 of the invention are preferably polymerized using photochemical, radical initiated, and other methods of polymerization, including methods known in the art to accelerate the polymerization of methacrylate monomers. The polymers show good mechanical properties, good adhesion to metals, glass and ceramics. Furthermore they show a relatively low water absorption and especially low shrinkage during the polymerization.

Compounds within the scope of general formula I are useful as dental adhesives, and are preferably used in dental compositions which include a self cure catalyst such as benzoyl peroxide. Polymerizable dental composite compositions in accordance with the invention preferably include at least one compound within the scope of general formula I and a filler, such as organic polymer particles and/or inorganic particles.

### Example 1

6.808 g (20.00 mmol) bisphenol-A diglycidyl ether, 1.462 g (10.00 mmol) adipic acid and 0.091 g triethylbenzylammoniumchloride are dissolved in one another and stirred and heated quickly to 150°C (2-5 minutes) and reacted for 1 hour at 90°C to form a reaction mixture. Then 0.009 g hydroquinone and 1.722 g (20.00 mmol) methacrylic acid are added to the reaction mixture, stirred and reacted for four hours at 90°C. The methacrylate terminated macromonomer obtained is soluble in organic solvents including chloroform, DMF and THF, and has Mₙ(vpo) of 1050 g/mol, T_{g} of 13.9°C the formula (C₅₆H₇₀O₁₆) 999.17 g/mol with a calculated C of 67.32 and H of 7.06 and a found C of 67.37 and H of 7.34.

### Example 2

6.808 g (20.00 mmol) bisphenol-A diglycidyl ether, 2.023 g (10.00 mmol) sebacic acid and 0.091 g triethylbenzylammoniumchloride were stirred while heating to 150°C (2-5 minutes) and allowed to react for 1 hour at 90°C to form a reaction mixture. Then 0.009 g hydroquinone and 1.722 g (20.00 mmol) methacrylic acid are added to the reaction mixture, dissolved with stirring and reacted for four hours at 90°C. The methacrylate terminated macromonomer obtained is soluble in organic solvents including chloroform, DMF and THF. It has Mₙ(vpo) of 1130 g/mol, T_{g} of 6.9°C and formula (C₆₀H₇₈O₁₆) 1055.27 g/mol.

### Example 3

6.808 g (20.00 mmol) bisphenol-A diglycidyl ether, 2.222 g (10.00 mmol) 3,6,9-trioxaundecane dicarboxylic acid and 0.091 g triethylbenzylammonium chloride are stirred while heating to 150°C (2-5 minutes) and reacted for 1 hour at 90°C to form a reaction mixture. Then 0.009 g hydroquinone and 1.722 g (20.00 mmol) methacrylic acid are added to the reaction mixture stirred and reacted for further four hours at 90°C. The methacrylate terminated macromonomer obtained is soluble in organic solvents including chloroform, DMF and THF. It has Mₙ(vpo) of 1090 g/mol, T_{g} of 14.4°C and the formula (C₅₈H₇₄O₁₉) 1075.21 g/mol.

### Example 4

13.616 g (40.00 mmol) bisphenol-A diglycidyl ether, 2.924 g (20.00 mmol) adipic acid, 3.444 g (40.00 mmol) methacrylic acid, 5.052 g triethyleneglycoldimethacrylate, 0.182 g triethylbenzylammoniumchloride and 0.040 g 2,6-di-tert-butyl-p-cresol are reacted for four hours at 90°C. The methacrylate terminated macromonomer obtained is soluble in organic solvents including chloroform, DMF and THF. Its IR-spectrum has absorption at 1720 cm⁻¹ indicating ester groups and no absorption of epoxide groups at 915 and 3050 cm⁻¹.

### Example 5

13.616 g (40.00 mmol) bisphenol-A diglycidyl ether, 3.323 g (20.00 mmol) isophthalic acid, 3.444 g (40.00 mmol) methacrylic acid, 5.052 g triethyleneglycoldimethacrylate 0.182 g triethylbenzylammoniumchloride and 0.040 g 2,6-di-tert-p-cresol are reacted for four hours at 90°C. The methacrylate terminated macromonomer obtained in soluble in organic solvents including chloroform, DMF and THF. Its IR-spectrum has absorption at 1720 cm⁻¹ indicating ester groups and no absorption of epoxide groups at 915 and 3050 cm⁻¹.

### Example 6

20.425 g (60.00) mmol) bisphenol-A diglycidyl ether, 5.848 g (40.00 mmol) adipic acid, 3.444 g (40.00 mmol) methacrylic acid, 7.430 triethyleneglycoldimethacrylate, 0.246 g triethylbenzylammoniumchloride and 0.060 g 2,6-di-tert.-butyl-p-cresol are reacted for four hours at 90°C. The methacrylate terminated macromonomer obtained is soluble in organic solvents such as chloroform, DMF and THF. Its IR-spectrum has absorption at 1720 cm⁻¹ and no absorption of epoxide groups at 915 and 3050 cm-1 was observed.

### Composite material

### Example 7

13.616 g (40.00 mmol) bisphenol-A diglycidyl ether, 2.924 g (20.00 mmol) adipic acid, 3.444 g (40.00 mmol) methacrylic acid, 5.052 g triethyleneglycoldimethacrylate, 0.182 g triethylbenzylammoniumchloride and 0.040 g 2,6-di-tert-butyl-p-cresol are reacted for four hours at 90°C to form a macromonomer containing liquid. The methacrylate terminated macromonomer obtained is soluble in organic solvents including chloroform, DMF and THF. Its IR-spectrum has absorption at 1720 cm⁻¹ indicating ester groups and no absorption of epoxide groups at 915 and 3050 cm⁻¹. 24 g of the macromonomer containing liquid is mixed with 75 g of APH glass, 0.5 g of propamine and 0.5 g of camphorquinone to form a polymerizable dental composite forming material. Visible light is directed onto this dental composite forming material which polymerizes to form a polymeric dental composite having a shrinkage of 1.27 % a compressive strength of 129.8 MPa, a compressive modulus of 1505.0 MPa, a flexural strength of 52.6 MPa, and a diametrial tensile strength of 34.6 MPa

### Example 8

51.060 g (0.15 mol) bisphenol-A diglycidyl ether, 10.110 g (0.05 mol) sebacic acid, 17.220 g (0.20 mol) methacrylic acid, 0.402 g triethylbenzylammoniumchloride and 0.164 g 2,6-di-tert.-butyl-p-cresol were reacted for four hours at 90°C. The Methacrylate terminated macromonomer obtained is soluble in organic solvents including chloroform, DMF and THF. Its IR-spectrum has absorption at 1720 cm⁻¹ indicating ester groups and no absorption of epoxide groups at 915 and 3050 cm⁻¹.
Mₙ(calc.) = 783.9 g/mol, Mₙ (vpo) = 750 g/mol

### Dental Restorative

### Example 9

15.36 g of the macromonomer formed an described in Example 6, 8.64 g of triethyleneglycol methacrylic acid (TEGMA), 75.00 g of AP.H. glass filler, 0.50 g of camphorquinone and 0.50 g of propamine are mixed to form a dental restorative material. Visible light is applied to the dental restorative material to form a polymeric dental restorative having a Compressive strength of 139 (MPa), Flexural strength of 53 (MPa), Diametral tensile strength of 35 (MPa), Barcol hardness (# 934) of 86 and Polymerization shrinkage of 1.3 %.

### Dental Root Canal filling material

### Example 10

21.25 g of the macromonomer formed as described in Example 6, 5.32 g of TEGMA, 6.64 g of ethoxylated Bis-GMA, 66.45 g of calcium tungstate, 0.17 g of propamine and 0.17 g of dibenzylperoxide are mixed to form a dental root canal filing material which polymerizes with a setting time at 23°C of 32 minutes setting time at 37°C of 18 minutes, solubility of 0.3 %, Radio-opacity of 9.2 mm Al and Polymerization shrinkage of 1.0 %.

## Claims

1. A dental material comprising: a filler and a α,ω-acrylate-terminated epoxidecarboxylic acid macromonomer compound formed by reaction of at least one diepoxide, at least one dicarboxylic acid and at least one unsaturated mono-carboxylic acid and having the general formula: 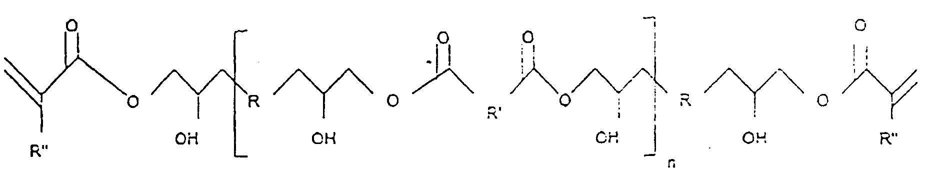
wherein R is a residue arisen from a diepoxide,
R' is a substituted or unsubstituted aliphatic, araliphatic, cycloaliphatic or aromatic moiety formed from a dicarboxylic acid,
R'' is hydrogen, a substituted or unsubstituted aliphatic, araliphatic, aromatic or cycloaliphatic moiety formed from a mono-carboxylic acid, and n is an integer from 1 to 20, said material polymerizing with a polymerization shrinkage of not more than 1.3 volume percent.

2. The dental material according to Claim 1
**characterized in that** it is a dental restorative, dental cement or dental luting material, or a dental root canal filling material or dental adhesive material.

3. The dental material of Claim 1 wherein R is

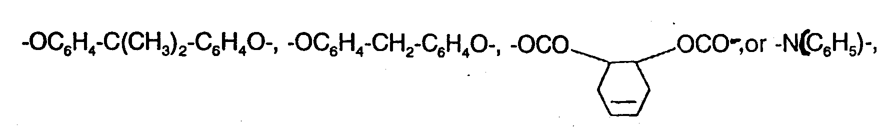

4. The dental material accoarding to claims 1 to 3
wherein R is an aromatic moiety formed from a diepoxide and R'' is methyl.

5. The dental material of one of claims 1 to 4 wherein R' is
-CH₂CH₂-, -CH₂CH₂CH₂CH₂-, -CH₂-,-(CH₂)₃-, -C₆H₄-, or -C₆H₁₀-.

6. The dental mateiral according to one of claims 1 to 5 wherein the filler is an organic filler or an inorganic filler.

## Patentansprüche

1. Dentalmaterial, umfassend einen Füllstoff sowie eine α,ω-acrylatterminierte Epoxidcarbonsäuremacromonomerverbindung, die erhältlich ist durch Umsetzung mindestens eines Diepoxids, mindestens einer Dicarbonsäure und mindestens einer ungesättigten Monocarbonsäure und die folgende allgemeine Folgel aufweist: 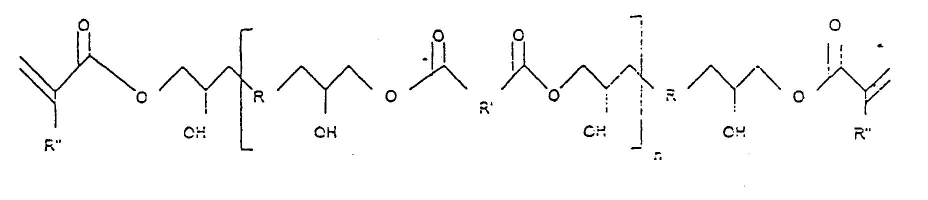
wobei R für einen aus einem Diepoxid stammenden Rest steht,
R' für eine aus einer Dicarbonsäure stammende substituierte oder unsubstituierte aliphatische, araliphatische, cycloaliphatische oder aromatische Gruppe steht,
R'' für Wasserstoff, eine aus einer Monocarbonsäure stammende substituierte oder unsubstituierte aliphatische, araliphatische, aromatische oder cycloaliphatische Gruppe steht und n für eine ganze Zahl von 1 bis 20 steht, wobei das Material mit einer Polymerisationsschrumpfung von nicht mehr als 1,3 Volumenprozent polymerisierbar ist.

2. Dentalmaterial nach Anspruch 1, dadurch gekennzeichnet, daß es ein dentales Restaurationsmaterial, ein Dentalzement oder ein dentales Dichtungsmaterial oder ein dentales Wurzelkanalfüllmaterial oder ein dentales Adhäsionsmaterial ist.

3. Dentalmaterial nach Anspruch 1, wobei R für 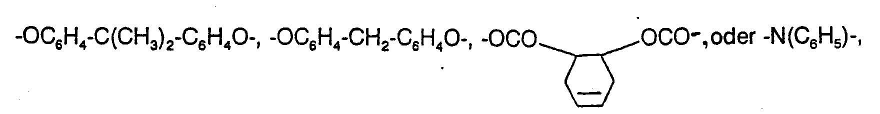 steht.

4. Dentalmaterial nach einem der Ansprüche 1 bis 3, wobei R für eine aus einem Diepoxid stammende aromatische Gruppe steht und R'' für Methyl steht.

5. Dentalmaterial nach einem der Ansprüche 1 bis 4, wobei R' für
-CH₂CH₂-, -CH₂CH₂CH₂CH₂-, -CH₂-,-(CH₂)₃-, -C₆H₄-, oder - C₆H₁₀-,
steht.

6. Dentalmaterial nach einem der Ansprüche 1 bis 5, wobei der Füllstoff ein organischer Füllstoff oder ein anorganischer Füllstoff ist.

## Revendications

1. Matériau dentaire comprenant une charge et un composé macromonomère d'époxyde-acide carboxylique à terminaison acrylate en α,ω, formé par réaction d'au moins un diépoxyde, d'au moins un acide dicarboxylique et d'au moins un acide monocarboxylique insaturé, et ayant la formule générale : 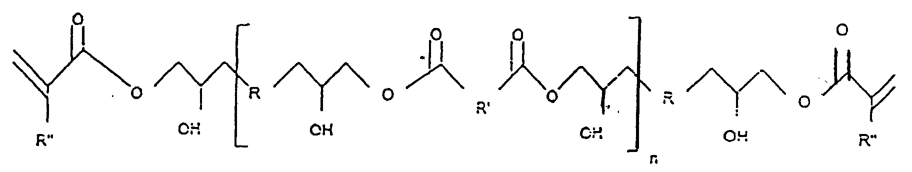
où R est un résidu provenant d'un diépoxyde,
R' est un motif aliphatique, araliphatique, cycloaliphatique ou aromatique, substitué ou non substitué, formé à partir d'un acide dicarboxylique,
R'' est l'hydrogène, un motif aliphatique, araliphatique, aromatique ou cycloaliphatique, substitué ou non substitué, formé à partir d'un acide monocarboxylique, et n est un nombre entier de 1 à 20,
ledit matériau étant polymérisé avec un retrait sous l'effet de la polymérisation ne dépassant pas 1,3 % en volume.

2. Matériau dentaire selon la revendication 1, caractérisé en ce qu'il s'agit d'un matériau de réparation dentaire, d'un ciment dentaire ou d'un matériau dentaire de scellement, ou d'un matériau dentaire pour l'obturation des canaux d'une racine ou d'un matériau dentaire adhésif.

3. Matériau dentaire selon la revendication 1, où R est

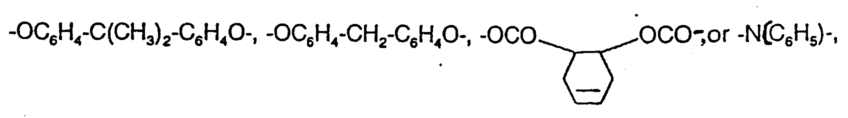

4. Matériau dentaire selon les revendications 1 à 3, où R est un motif aromatique formé à partir d'un diépoxyde, et R'' est un groupe méthyle.

5. Matériau dentaire selon l'une des revendications 1 à 4, où R' est -CH₂CH₂-, -CH₂CH₂CH₂CH₂-, -CH₂-, -(CH₂)₃-, -C₆H₄- ou -C₆H₁₀-.

6. Matériau dentaire selon l'une des revendications 1 à 5, dans lequel la charge est une charge organique ou une charge inorganique.
